(19) Europäisches Patentamt
European Patent Office
Office européen des brevets

(11) **EP 3 138 481 B1**

(12) **EUROPEAN PATENT SPECIFICATION**

(45) Date of publication and mention
of the grant of the patent:
**17.04.2019 Bulletin 2019/16**

(51) Int Cl.:
***A61B 5/00*** *(2006.01)*  ***A61B 8/00*** *(2006.01)*
***G10K 15/04*** *(2006.01)*

(21) Application number: **16186935.9**

(22) Date of filing: **02.09.2016**

(54) **OBJECT INFORMATION ACQUIRING APPARATUS AND CONTROL METHOD FOR OBJECT INFORMATION ACQUIRING APPARATUS**

OBJEKTINFORMATIONSERFASSUNGSVORRICHTUNG UND STEUERUNGSVERFAHREN FÜR OBJEKTINFORMATIONSERFASSUNGSVORRICHTUNG

APPAREIL D'ACQUISITION D'INFORMATIONS D'OBJET ET PROCÉDÉ DE COMMANDE D'APPAREIL D'ACQUISITION D'INFORMATIONS D'OBJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2015 US 201562214350 P**

(43) Date of publication of application:
**08.03.2017 Bulletin 2017/10**

(73) Proprietor: **Canon Kabushiki Kaisha**
**Tokyo 146-8501 (JP)**

(72) Inventors:
• **MIYASATO, Takuro**
**Tokyo, Tokyo 146-8501 (JP)**
• **FUKUTANI, Kazuhiko**
**Tokyo, Tokyo 146-8501 (JP)**
• **MASAKI, Fumitaro**
**Tokyo, Tokyo 146-8501 (JP)**
• **SUEHIRA, Nobuhito**
**Tokyo, Tokyo 146-8501 (JP)**
• **KRUGER, Robert A.**
**Oriental, North Carolina 28571 (US)**

(74) Representative: **TBK**
**Bavariaring 4-6**
**80336 München (DE)**

(56) References cited:
**US-A1- 2005 131 289    US-A1- 2013 031 982**
**US-A1- 2013 190 594    US-A1- 2013 190 595**
**US-A1- 2013 245 420**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present invention relates to an object information acquiring apparatus which acquires information of the inside of an object and to a control method thereof.

Description of the Related Art

A technique referred to as photoacoustic tomography (PAT) is recently being proposed as an optical imaging technique. When a living organism that is an object is irradiated with light such as pulsed laser light, an acoustic wave is generated as the light is absorbed by living tissue inside the object. This phenomenon is referred to as a photoacoustic effect and an acoustic wave generated by the photoacoustic effect is referred to as a photoacoustic wave. Since tissues constituting the object have respectively different absorption rates of optical energy, sound pressure is also different between the generated photoacoustic waves. With PAT, by detecting a generated photoacoustic wave with a probe and mathematically analyzing a detected signal, the distribution of optical characteristics inside the object, particularly, optical energy absorption density can be imaged.

In addition, an ultrasonic wave imaging technique is also known in which an ultrasonic wave is transmitted to an object and an ultrasonic wave transmitted through or reflected by the inside of the object is received to acquire morphological information on the inside of the object.

Methods combining these techniques are proposed in Patent Literature 1 (PTL 1) and Non-Patent Literature 1 (NPL 1). Specifically, both an object and an absorbing member placed near the object are simultaneously irradiated with light and a first photoacoustic wave generated inside the object and a second photoacoustic wave generated by the absorbing member and reflected inside the object are respectively measured. An image similar to PAT can be generated from a detected signal of the first photoacoustic wave and an image similar to ultrasonic wave imaging can be generated from a detected signal of the second photoacoustic wave. Hereinafter, a detected signal of the first photoacoustic wave will be referred to as a PAT signal and a detected signal of the second photoacoustic wave will be referred to as a photoacoustic-induced ultrasound signal (a PAUS signal). In addition, an image of the inside of the object which is reconstructed using a PAT signal will be referred to as a PAT image and an image of the inside of the object which is reconstructed using a PAUS signal will be referred to as a PAUS image.

Patent Literature 1: US Patent Application Publication No. 2010/0041987

Non-Patent Literature 1: Thomas Felix Fehm, Xose Luis Dean-Ben and Daniel Razansky, "Hybrid optoacoustic and ultrasound imaging in three dimensions and real time by optical excitation of a passive element", Proc. of SPIE Vol. 9323

Prior art which is related to this field of technology can be found in e.g. document US 2013/0190595 A1 disclosing a laser optoacoustic ultrasonic imaging system (louis) and methods of use, in document US 2013/0190594 A1 disclosing a scanning optoacoustic imaging system with high resolution and improved signal collection efficiency, in document US 2005/0131289 A1 disclosing an ultrasonic transducer probe, in document US 2013/0031982 A1 disclosing a photoacoustic apparatus and method for controlling the same, and in document US 2013/0245420 A1 disclosing a subject information acquiring device and subject information acquiring method.

SUMMARY OF THE INVENTION

Using the methods proposed in PTL 1 and NPL 1 enables a PAT image and a PAUS image to be acquired with one apparatus. However, with a configuration in which an object and an absorbing member are simultaneously irradiated with light as in the methods proposed in PTL 1 and NPL 1, a first photoacoustic wave generated inside the object and a second photoacoustic wave generated by the absorbing member end up being mixed up. As a result, there is a problem that an artifact caused by a PAUS signal is introduced into a PAT image and an artifact caused by a PAT signal is introduced into a PAUS image and, consequently, causing a decline in visibility of an image of the inside of an object.

The present invention has been made in consideration of the circumstances described above and provides a technique that enables both measurement of a photoacoustic wave generated inside an object and measurement of a photoacoustic wave generated by an absorbing member to be accurately performed.

The present invention provides an object information acquiring apparatus as specified in the claims.

Using the present invention enables both measurement of a photoacoustic wave generated inside an object and measurement of a photoacoustic wave generated by an absorbing member to be accurately performed.

Further features of the present invention will become apparent from the following description of exemplary embodiments with reference to the attached drawings.

BRIEF DESCRIPTION OF THE DRAWINGS

FIGS. 1A and 1B are system configuration diagrams of a photoacoustic measuring apparatus according to an embodiment;

FIG. 2 is a processing flow chart of a photoacoustic measuring apparatus according to an embodiment;

FIGS. 3A and 3B are system configuration diagrams of a photoacoustic measuring apparatus according to a first practical example;

FIGS. 4A and 4B are system configuration diagrams of a photoacoustic measuring apparatus according to the first practical example;

FIGS. 5A and 5B are system configuration diagrams of a photoacoustic measuring apparatus according to the first practical example;

FIGS. 6A and 6B are system configuration diagrams of a photoacoustic measuring apparatus according to a second practical example (not claimed);

FIGS. 7A and 7B are system configuration diagrams of a photoacoustic measuring apparatus according to the second practical example (not claimed);

FIGS. 8A and 8B are system configuration diagrams of a photoacoustic measuring apparatus according to the second practical example (not claimed);

FIGS. 9A and 9B are system configuration diagrams of a photoacoustic measuring apparatus according to the second practical example (not claimed);

FIGS. 10A and 10B are system configuration diagrams of a photoacoustic measuring apparatus according to a third practical example (not claimed); and

FIGS. 11A and 11B are system configuration diagrams of a photoacoustic measuring apparatus according to a fourth practical example (not claimed) .

DESCRIPTION OF THE EMBODIMENTS

Hereinafter, a preferred embodiment of the present invention and examples useful for unstanding the present invention will be described with reference to the drawings.

However, it is to be understood that dimensions, materials, shapes, relative arrangements, and the like of components described below are intended to be changed as deemed appropriate in accordance with configurations and various conditions of apparatuses to which the present invention is to be applied and are not intended to limit the scope of the invention to the description presented below.

The present invention relates to a technique for detecting an acoustic wave propagating from an object and generating and acquiring specific information of the inside of the object. Accordingly, the present invention can be considered an object information acquiring apparatus or a control method thereof, or an object information acquiring method and a signal processing method (not claimed). An example useful for understanding the present invention is a program that causes an information processing apparatus including hardware resources such as a CPU to execute these methods or a storage medium storing the program.

The present invention is applied to an object information acquiring apparatus using photoacoustic tomography technology in which an object is irradiated with light (an electromagnetic wave) and an acoustic wave generated at and propagating from a specific position inside the object or on a surface of the object according to a photoacoustic effect is received (detected). Since such an apparatus obtains specific information of the inside of an object in a format such as image data or characteristic distribution information based on photoacoustic measurement, the apparatus can also be called a photoacoustic measuring apparatus, a photoacoustic imaging apparatus, a photoacoustic image forming apparatus, or simply a photoacoustic apparatus.

Specific information in a photoacoustic apparatus includes a distribution of generation sources of acoustic waves generated due to light irradiation, a distribution of initial sound pressure inside an object, a distribution of optical energy absorption density or a distribution of absorption coefficients derived from a distribution of initial sound pressure, and a distribution of concentration of substances constituting tissue. Concentration of substances refers to oxygen saturation, oxyhemoglobin concentration, deoxyhemoglobin concentration, total hemoglobin concentration, and the like. Total hemoglobin concentration is a sum of oxyhemoglobin concentration and deoxyhemoglobin concentration. A distribution of fat, collagen, or water, and the like may also be considered. In addition, specific information may be obtained as distribution information at respective positions inside the object instead of as numerical data. In other words, distribution information such as a distribution of absorption coefficients and a distribution of oxygen saturation can be adopted as object information.

The present invention is also applied to an apparatus using ultrasonic imaging technology in which an acoustic wave (an ultrasonic wave) is transmitted to an object and an acoustic wave having propagated through (or reflected, scattered, or transmitted by) the inside of the object is received to acquire object information as image data. In the case of an apparatus using ultrasonic imaging technology, the acquired object information is information reflecting a difference in acoustic impedances among tissues inside the object.

An acoustic wave according to the present invention is typically an ultrasonic wave and includes an elastic wave which is also referred to as a sonic wave or an acoustic wave. An acoustic wave generated by a photoacoustic effect is referred to as a photoacoustic wave or an optical ultrasonic wave. An electrical signal (a received signal) converted from an acoustic wave by a probe is also referred to as an acoustic signal, and an acoustic signal derived from a photoacoustic wave is particularly referred to as a photoacoustic signal.

As an object to which the present invention is applied, a breast of a living organism can be assumed. Therefore, the present invention can be assumed to be used when examining a lesion (such as breast cancer) of the breast. However, the object is not limited thereto and other parts of a living organism or a non-living material can also be examined.

First embodiment

<System Configuration>

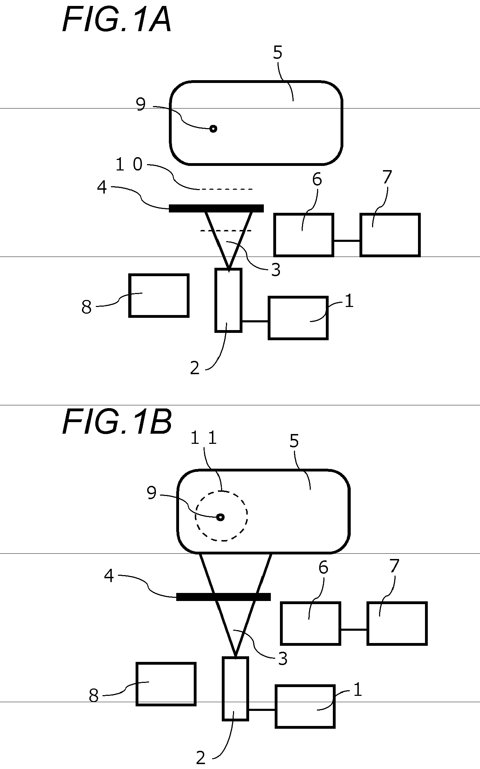

A configuration of a photoacoustic measuring apparatus according to the present embodiment will be described with reference to FIGS. 1A and 1B. The photoacoustic measuring apparatus according to the present embodiment includes a light source 1, an irradiating unit 2, an acoustic wave detector 6, a signal processing unit 7, a switching unit 8, and an acoustic wave generating member 4. Reference numeral 3 denotes light emitted from the irradiating unit 2. Reference numeral 5 denotes a part of a living organism that is an object and reference numeral 9 denotes a light absorber existing inside the object 5. Reference numeral 11 denotes a first photoacoustic wave generated inside the object 5 (by the light absorber 9) and reference numeral 10 denotes a second photoacoustic wave generated by the acoustic wave generating member 4.

Hereinafter, an outline of a measurement method will be presented while describing respective means that constitute the photoacoustic measuring apparatus according to the present embodiment.

<<Light source>>

The light source 1 is an apparatus that generates measurement light 3 used in photoacoustic measurement. Pulsed light is used as the measurement light. While the light source 1 is desirably a laser light source for the purpose of obtaining a large output, a light-emitting diode, a flash lamp, or the like may be used in place of a laser. When using a laser as the light source 1, various lasers such as a solid-state laser, a gas laser, a dye laser, and a semiconductor laser can be used.

Ideally, a Nd:YAG-excited Ti:Sa laser or alexandrite laser with high output and continuously variable wavelength is used. Alternatively, single-wavelength lasers with different wavelengths may be provided in plurality.

Timings, waveforms, intensity, and the like of irradiation of the pulsed light 3 are controlled by a light source controller (not shown). The light source controller may be integrated with the light source 1.

In addition, desirably, a wavelength of the pulsed light 3 is a specific wavelength which is absorbed by a specific component among components constituting the object 5 and which enables light to propagate to the inside of the object 5. The wavelength of light is selected in accordance with light-absorbing substances in a living organism that is a measurement object. Examples of light-absorbing substances include oxygenated hemoglobin, deoxygenated hemoglobin, a blood vessel containing oxygenated hemoglobin or deoxygenated hemoglobin in a large amount, and a malignant tumor containing a large number of new blood vessels. Glucose, cholesterol, and the like may also be considered light-absorbing substances. Specifically, when the object 5 is a living organism, light with a wavelength of 700 nm or more and 1100 nm or less is favorably used.

Furthermore, in order to effectively generate a photoacoustic wave, light is favorably irradiated in a significantly short period of time in accordance with thermal characteristics of the object 5. When the object 5 is a living organism, a pulse width of the pulsed light 3 generated by the light source 1 is preferably around 10 to 50 nanoseconds. Hereinafter, the pulsed light 3 generated by the light source 1 may also be referred to as measurement light 3 or, simply, light 3.

<<Irradiating unit>>

The irradiating unit 2 is an irradiating optical system for irradiating the object 5 or the acoustic wave generating member 4 with pulsed light 3 from the light source 1. Typically, the irradiating unit 2 guides the pulsed light 3 to the object 5 while processing the pulsed light 3 into a desired distribution shape using optical parts such as a mirror which reflects light, a lens which enlarges light, and a diffuser plate which diffuses light. The irradiating unit 2 can also propagate the pulsed light 3 using, for example, an optical waveguide such as an optical fiber. Any optical part may be used as long as light emitted by the light source 1 can be irradiated in a desired shape. Spreading light over a relatively wide area is more favorable than focusing light with a lens from the perspectives of safety with respect to the object 5 and expanding a diagnostic area. In addition, a moving mechanism of the irradiating unit 2 may be provided to enable positions where light is irradiated to be changed.

<<Acoustic wave detector>>

The acoustic wave detector 6 is an apparatus which detects an acoustic wave propagating from the object 5 and converts the acoustic wave into an electrical signal. As acoustic waves propagating from the object 5, a first acoustic wave 11 generated inside the object 5 due to irradiation of the pulsed light 3 and a second acoustic wave 10 generated by the acoustic wave generating member 4 due to irradiation of the pulsed light 3 and transmitted through or reflected by the inside of the object 5 are assumed. The acoustic wave detector 6 is also referred to as a probe, a detector, a photoacoustic wave detector, and a transducer.

Since acoustic waves generated by a living organism are ultrasonic waves from 100 KHz to 100 MHz, an ultrasonic wave detector capable of detecting this frequency band is favorably used as the acoustic wave detector 6. Specifically, a transducer using a piezoelectric phenomenon, a transducer using optical resonance, a transducer using a variation in capacity, or the like can be used. In addition, desirably, the acoustic wave detector 6 has a high receiving sensitivity and a wide frequency band.

Furthermore, the acoustic wave detector 6 may have a plurality of detecting elements arranged one-dimensionally or two-dimensionally and may be configured to be movable by a scanning mechanism. Since the use of multidimensionally-arranged elements enables acoustic waves to be simultaneously detected at a plurality of locations, measurement time can be shortened and an effect of vibration of the object 5 and the like can be reduced. In addition, a single element focused by an acoustic lens may be used. Furthermore, the acoustic wave detector 6 includes a receiving circuit which amplifies an obtained electrical signal and converts the electrical signal into a digital signal. Specifically, the acoustic wave detector 6 includes an amplifier, an A/D converter, an FPGA chip, and the like.

Moreover, when a plurality of detected signals are obtained, desirably, a plurality of signals can be processed simultaneously. Accordingly, a period of time until an image is formed can be reduced. In addition, acoustic wave signals detected at a same position with respect to the object 5 can be integrated to create a single signal. A method of integration may involve adding up the signals or obtaining an average of the signals. Alternatively, the signals may be respectively weighted and then added up. Moreover, a "detected signal" as used in the present specification is a concept including both an analog signal output from an acoustic wave detector and a digital signal obtained by subsequent A/D conversion.

<<Signal processing unit>>

The signal processing unit 7 is an apparatus which processes an electrical signal (a digital signal) obtained by the acoustic wave detector 6 and which reconstructs an image representing optical characteristics and morphological information of the inside of the object. While methods of reconstruction include a Fourier transform method, a universal back-projection method (UBP method), and a filtered back-projection method, any method may be used. The generated image is presented to a user by a display apparatus (not shown) .

Moreover, the signal processing unit 7 may be an independent computer including a CPU (processor), a main storage device, and an auxiliary storage device or may be exclusively-designed hardware. Typically, a work station or the like is used and the processes described above are performed by software (a computer program).

<<Acoustic wave generating member>>

The acoustic wave generating member 4 is a member which absorbs light and generates an acoustic wave (a second acoustic wave) 10. Since the acoustic wave 10 generated by the acoustic wave generating member 4 is applied to (transmitted to) the object 5, the acoustic wave generating member 4 is arranged in a vicinity of the object 5. The acoustic wave generating member 4 may be any member as long as light is absorbed and a photoacoustic wave is generated. The acoustic wave generating member 4 may have a dot shape, a linear shape, a plate-like shape, or any shape as long as the shape enables a generated photoacoustic wave to propagate to the object 5. However, the acoustic wave generating member 4 favorably has a sheet shape or a flat plate shape. This is because a sheet shape or a flat plate shape causes the acoustic wave 10 generated by the acoustic wave generating member 4 to be a planar wave and, accordingly, attenuation with respect to propagation distance is smaller (as compared to a spherical acoustic wave generated by a point sound source).

While dependent on a configuration of the switching unit 8 to be described later, the acoustic wave generating member 4 may be an absorbing-type polarizing plate which only absorbs light in a specific polarization direction and which transmits other light or a light absorber which only absorbs light with a specific wavelength and which transmits other light. In addition, the acoustic wave generating member 4 may be movable to a first position which is retreated from an optical path between the irradiating unit 2 and the object 5 and to a second position which is inserted to the optical path so as to block light to the object 5.

<<Switching unit>>

The switching unit 8 is means or a mechanism for switching a measurement mode of the photoacoustic measuring apparatus. The photoacoustic measuring apparatus according to the present embodiment has at least a first mode shown in FIG. 1B and a second mode shown in FIG. 1A. The first mode is a mode in which a PAT signal is acquired by irradiating the object 5 with the pulsed light 3 and detecting the first acoustic wave 11 generated inside the object 5 with the acoustic wave detector 6 and is also referred to as a PAT measurement mode. The second mode is a mode in which a PAUS signal is acquired by irradiating the acoustic wave generating member 4 with the pulsed light 3 and detecting the second acoustic wave 10 generated by the acoustic wave generating member 4 and having propagated inside the object 5 with the acoustic wave detector 6 and is also referred to as a PAUS measurement mode. In the first mode, the switching unit 8 may prevent light from being absorbed (prevent the second acoustic wave 10 from being generated) by the acoustic wave generating member 4 and, in the second mode, the switching unit 8 may prevent light from being absorbed (prevent the first acoustic wave 11 from being generated) by the object 5. By switching between modes in this manner, mixing of a PAT signal and a PAUS signal can be prevented.

For example, the switching unit 8 may switch targets to be irradiated with the light 3 so that only the object 5 is irradiated with the light 3 in the first mode and only the acoustic wave generating member 4 is irradiated with the light 3 in the second mode. Switching of targets to be irradiated with the light 3 may be realized by any method such as moving the object 5 (not claimed) or the acoustic wave generating member 4 and moving or changing (not claimed) the irradiating unit 2 or an optical path. In addition, the switching unit 8 may switch targets which absorb the light 3 so that the light 3 is absorbed only by the object 5 in the first mode and the light 3 is absorbed only by the acoustic wave generating member 4 in the second mode. Switching of targets which absorb the light 3 may be realized by any method such as moving the object 5 (not claimed) or the acoustic wave generating member 4, moving or changing (not claimed) the irradiating unit 2 or an optical path, switching the polarization direction or wavelength of the light 3 (not claimed), and switching the characteristic of the acoustic wave generating member 4 (not claimed).

Specifically, when the acoustic wave generating member 4 is movable to a position which is retreated from the optical path between the irradiating unit 2 and the object 5 and to a position which blocks the optical path, the switching unit 8 may be a mechanism for switching the position of the acoustic wave generating member 4. When the irradiating unit 2 is movable to a position where only the object 5 is irradiated and to a position where only the acoustic wave generating member 4 is irradiated, the switching unit 8 may be a mechanism for switching the position of the irradiating unit 2. When the irradiating unit 2 has an irradiation port for only irradiating the object 5 and an irradiation port for only irradiating the acoustic wave generating member 4, the switching unit 8 may be a mechanism for switching the irradiation port from which the light 3 is to be output (not claimed). When the acoustic wave generating member 4 is a polarizing plate which only absorbs light in a specific polarization direction, the switching unit 8 may be a mechanism which switches the polarization direction of the pulsed light 3 irradiated by the irradiating unit 2 (not claimed). When the acoustic wave generating member 4 is a member with a property of only absorbing light with a specific wavelength, the switching unit 8 may be a mechanism which switches the wavelength of the pulsed light 3 irradiated from the irradiating unit 2 (not claimed).

<<Method of measuring object>>

A method of measuring a living organism that is an object with the photoacoustic measuring apparatus according to the present embodiment will now be described.

(First mode)

In the first mode, as shown in FIG. 1B, the light 3 emitted from the light source 1 passes through the irradiating unit 2 and irradiates the object 5. The light 3 having entered the object 5 attenuates as the light 3 is diffused and absorbed inside the object (when the object 5 is a living organism, inside living tissue) and forms a light quantity distribution in accordance with a distance from an irradiation position and the like.

When a part of energy of the light 3 having propagated inside the living organism is absorbed by the light absorber 9 that is blood or the like, the acoustic wave 11 is generated by the light absorber 9 due to thermal expansion. For example, when cancer exists in the living organism, light is specifically absorbed by new blood vessels in the cancer in a similar manner to blood vessels in other healthy parts and the acoustic wave 11 is generated.

The generated acoustic wave 11 propagates inside the object 5 and is detected by the acoustic wave detector 6 and converted into an analog first electrical signal. Moreover, the acoustic wave detector 6 according to the present embodiment has a large number of acoustic wave detecting elements (not shown) so that a position where an acoustic wave is generated can be specified. The acoustic wave detector 6 amplifies and digitally converts the first electrical signal and outputs a first detected signal (a PAT signal). The first detected signal is stored in a memory (not shown) inside the signal processing unit 7.

(Second mode)

In the second mode, as shown in FIG. 1A, the light 3 emitted from the light source 1 passes through the irradiating unit 2 and irradiates the acoustic wave generating member 4. Upon absorbing energy of the light 3, the acoustic wave generating member 4 generates the acoustic wave 10 due to thermal expansion.

The generated acoustic wave 10 reaches the object 5 and propagates inside the object 5. The acoustic wave 10 reflected, scattered, or transmitted inside the object 5 is detected by the acoustic wave detector 6 and converted into an analog second electrical signal. The acoustic wave detector 6 amplifies and digitally converts the second electrical signal and outputs a second detected signal (a PAUS signal). The second detected signal is stored in the memory (not shown) inside the signal processing unit 7.

<Method of acquiring object information>

Next, an outline of a process for calculating first object information from the first detected signal stored in the signal processing unit 7 will be described. The signal processing unit 7 uses the first detected signal to obtain a distribution of initial sound pressure inside the object 5 according to the UBP method. In addition, the signal processing unit 7 may obtain a distribution of absorption coefficients inside the object 5 based on the distribution of initial sound pressure and light quantity distribution. Furthermore, the signal processing unit 7 may use spectral information to calculate oxygen saturation or a distribution of glycogen concentration from the distribution of absorption coefficients of light with other wavelengths. An image representing these distributions is referred to as a PA image.

An outline of a process for calculating second object information from the second detected signal stored in the signal processing unit 7 will be described. The second detected signal includes an acoustic wave directly incident to the acoustic wave detector 6 from the acoustic wave generating member 4 and an acoustic wave generated by the acoustic wave generating member 4 and incident to the acoustic wave detector 6 after being reflected and scattered inside the object. The following equations represent an example of an image reconstruction method using the second detected signal.

[Math. 1]

$$R(r) = \sum_{i}^{N} b\left( r_i, \frac{|r_i - r| + |r_i - r_a|}{c} \right)$$

$$b(r_i, t) = 2p(r_i, t) - 2t\frac{\partial p(r_i, t)}{\partial t}$$

In the equations, $r_i$ denotes a position of a detecting element, N denotes the number of elements, $r_a$ denotes a position of the acoustic wave generating member 4, and c denotes acoustic wave velocity. $p(r_i, t)$ denotes sound pressure received by a detecting element at a position $r_i$ during a period of time t.

<Processing flow>

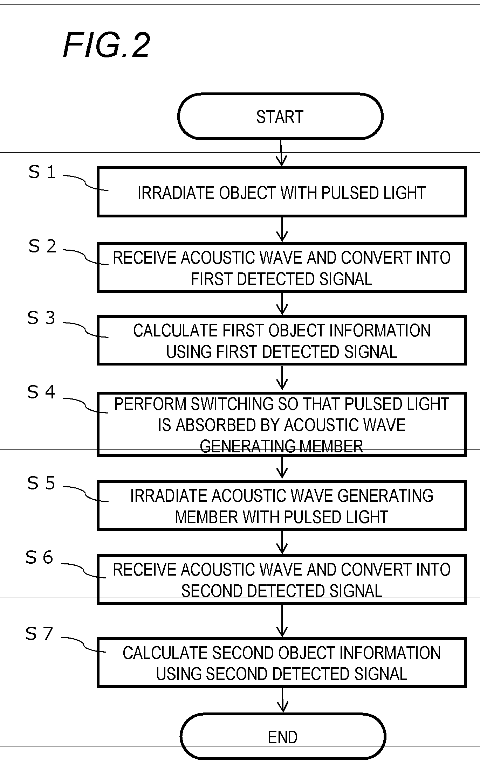

An operation example for realizing the processes described above will now be described with reference to FIG. 2. FIG. 2 is a flow chart of processes executed by the photoacoustic measuring apparatus according to the present embodiment. Steps S1 and S2 in FIG. 2 correspond to a first measurement step in the first mode, step S4 corresponds to a switching step, and steps S5 and S6 correspond to a second measurement step in the second mode.

First, in step S1, the irradiating unit 2 irradiates the object 5 with the pulsed light 3 from the light source 1. Accordingly, due to a photoacoustic effect, the first acoustic wave 11 is generated inside the object 5.

Next, in step S2, the acoustic wave detector 6 receives the first acoustic wave 11 and outputs a first detected signal. The first detected signal is stored in a memory included in the signal processing unit 7. Moreover, when irradiation of the pulsed light 3 is performed a plurality of times, irradiation of the pulsed light 3 and signal acquisition (steps S1 and S2) are repetitively executed. Therefore, timings of irradiation of the pulsed light 3 and detection of an acoustic wave must be synchronized.

Next, in step S3, the signal processing unit 7 calculates first object information using the first detected signal. At this point, a distribution of initial sound pressure or a distribution of absorption coefficients inside the object is to be calculated as object information.

Next, in step S4, the switching unit 8 switches the mode of the photoacoustic measuring apparatus from the first mode to the second mode. Specifically, the photoacoustic measuring apparatus is switched to a state where the pulsed light 3 is absorbed by the acoustic wave generating member 4 and the second acoustic wave 10 is output from the acoustic wave generating member 4. As described earlier, any method may be adopted to switch the modes.

Next, in step S5, the irradiating unit 2 irradiates the acoustic wave generating member 4 with the pulsed light 3. Accordingly, due to a photoacoustic effect, the second acoustic wave 10 is generated by the acoustic wave generating member 4. The second acoustic wave 10 is transmitted to the object 5 and is reflected or scattered by tissue or structures inside the object 5.

Next, in step S6, the acoustic wave detector 6 receives the second acoustic wave 10 having been reflected or scattered inside the object 5 and outputs a second detected signal. The second detected signal is stored in the memory included in the signal processing unit 7. Moreover, when irradiation of the pulsed light 3 is performed a plurality of times, irradiation of the pulsed light 3 and signal acquisition (steps S5 and S6) are repetitively executed. Therefore, timings of irradiation of the pulsed light 3 and detection of an acoustic wave must be synchronized.

Next, in step S7, the signal processing unit 7 calculates second object information using the second detected signal. At this point, a distribution of reflection of acoustic waves inside the object is to be calculated as object information.

While the first object information is calculated immediately after acquiring the first detected signal and the second object information is calculated immediately after acquiring the second detected signal in this case, alternatively, the first object information and the second object information may be collectively calculated after acquiring both the first detected signal and the second detected signal.

In addition, the acoustic wave generating member 4 may be irradiated with the pulsed light 3 first to acquire the second detected signal and the object 5 may be subsequently irradiated with the pulsed light 3 to acquire the first detected signal. In this case, the process of step S4 becomes a process of switching from the second mode to the first mode or, in other words, a process (step S4') of making a switch so that the pulsed light 3 is absorbed by the object 5. Furthermore, the first mode and the second mode may be alternatively repeated. In this case, after alternately repeating acquisition and accumulation of the first detected signal and acquisition and accumulation of the second detected signal in an order of S1 → S2 → S4 → S5 → S6 → S4' → S1 → S2 → S4 → S5 → ..., the first object information and the second object information may be ultimately calculated.

With the configuration according to the present embodiment described above, by having the switching unit 8 switch between the first mode and the second mode, the first acoustic wave 11 from the object 5 and the second acoustic wave 10 from the acoustic wave generating member 4 occur at different timings (do not occur simultaneously). Therefore, the first detected signal based on the first acoustic wave 11 and the second detected signal based on the second acoustic wave 10 are never mixed in signals detected by the acoustic wave detector 6. As a result, SN ratios of both the first detected signal and the second detected signal can be improved and object information with higher visibility as compared to conventional methods can be obtained.

<First practical example>

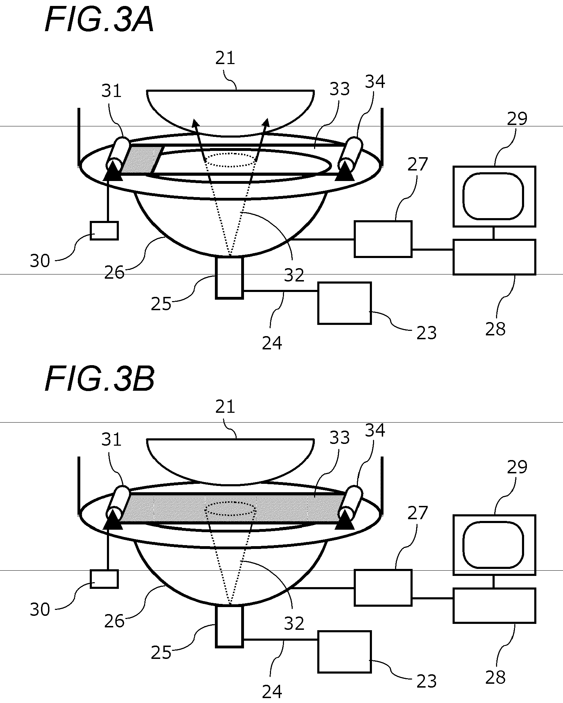

FIGS. 3A and 3B are system configuration diagrams of a photoacoustic measuring apparatus according to a first practical example. FIG. 3A shows a state of the first mode (PAT measurement) and FIG. 3B shows a state of the second mode (PAUS measurement).

The photoacoustic measuring apparatus according to the first practical example includes an acoustic wave detector made up of a bowl-shaped probe 26 and a receiving circuit 27. The probe 26 is configured such that a plurality of cMUT (capacitive micro-machined ultrasonic transducer) elements are arranged along an inside surface of a hemisphere.

A light source 23 is a Nd:YAG-excited Ti:Sa laser light source capable of irradiating light with a pulse width of 30 nanoseconds at 10 Hz. The pulsed light has a wavelength of 797 nm. Light exiting the Ti:Sa laser light source passes through an optical fiber 24 and is sent to an irradiating optical system 25 that is an irradiating unit, and emitted as pulsed light 32 through a lens and a diffuser plate toward an opening of the bowl-shaped probe 26 from a center of a bottom of the probe 26.

A polyethylene sheet is stretched across the opening of the probe 26 by rollers 31 and 34. The polyethylene sheet is constituted by a transparent sheet section 33 which transmits light with a wavelength of 797 nm and a black sheet section 22 in which black ink that completely absorbs light with a wavelength of 797 nm is mixed. The black sheet section 22 corresponds to an acoustic wave generating member. The switching unit 30 is an apparatus which varies a position of the black sheet section 22 by rotating the rollers 31 and 34 to wind the polyethylene sheet.

(First mode)

In the first mode, the switching unit 30 rolls the rollers 31 and 34 clockwise in FIG. 3A to wind the black sheet section 22 with the roller 31 and causes the black sheet section 22 to retreat from an optical path between the irradiating optical system 25 and an object 21. When the pulsed light 32 is irradiated in this state, the pulsed light 32 is transmitted through the transparent sheet section 33 of the polyethylene sheet and irradiated on the object 21. The first acoustic wave generated inside the object is detected by the probe 26. Detected sound pressure is converted into an electrical signal. The detected sound pressure converted into the electrical signal is amplified by an amplifier in the receiving circuit 27, converted into digital data, and output as a first detected signal. The first detected signal is stored in a memory of a work station PC 28 that is a signal processing unit. The work station PC 28 executes a program of the UBP method and converts the first detected signal into a distribution of absorption coefficients that is first object information. The calculated distribution of absorption coefficients is displayed on a liquid crystal monitor 29.

(Second mode)

In the second mode, the switching unit 30 rolls the rollers 31 and 34 counter-clockwise in FIG. 3B to wind the transparent sheet section 33 with the roller 34 and inserts the black sheet section 22 to the optical path between the irradiating optical system 25 and the object 21. At this point, the optical path is totally blocked by the black sheet section 22 so that the pulsed light 32 completely misses the object 21.

When the pulsed light 32 is irradiated in this state, the pulsed light 32 is irradiated on the black sheet section 22 and absorbed by the black sheet section 22. A part of the second acoustic wave generated by the black sheet section 22 is sent to the object 21, reflected or scattered inside the object 21, and detected by the probe 26. Detected sound pressure is converted into an electrical signal. The detected sound pressure converted into the electrical signal is amplified by an amplifier in the receiving circuit 27, converted into digital data, and output as a second detected signal. The second detected signal is stored in the memory of the work station PC 28. The work station PC 28 executes the calculation program represented by expression 1 and converts the second detected signal into an ultrasonic image that is second object information. The calculated ultrasonic image is displayed on the liquid crystal monitor 29.

(Modifications)

In the first practical example, by moving a position of the black sheet section 22 that is an acoustic wave generating member with the rollers 31 and 34, the optical path is switched between an open state (irradiation of the object with the light) and a blocked state (irradiation of the acoustic wave generating member with the light). However, this configuration is simply an example and the optical path may be switched between an open state and a blocked state by other configurations.

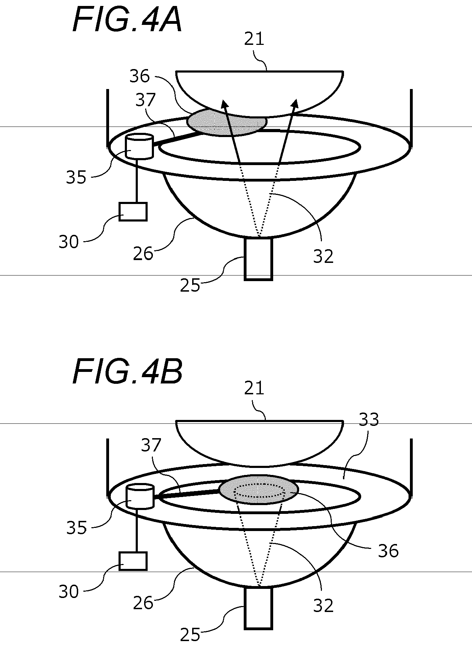

For example, a configuration shown in FIGS. 4A and 4B uses an acoustic wave generating member 36 made of a material (such as a black polyethylene sheet) which absorbs light. The acoustic wave generating member 36 is attached to a tip of a swingable arm 37 and is movable between a position (FIG. 4A) which is retreated from the optical path between the irradiating optical system 25 and the object 21 and a position (FIG. 4B) which blocks the optical path. In the first mode, by controlling a motor 35 to change an angle of the arm 37, the switching unit 30 moves the acoustic wave generating member 36 to the retreated position and causes the object 21 to be irradiated with the pulsed light 32. On the other hand, in the second mode, the switching unit 30 moves the acoustic wave generating member 36 to the blocking position and causes the pulsed light 32 to be absorbed by the acoustic wave generating member 36. Even with this configuration, the optical path can be switched between an open state and a blocked state.

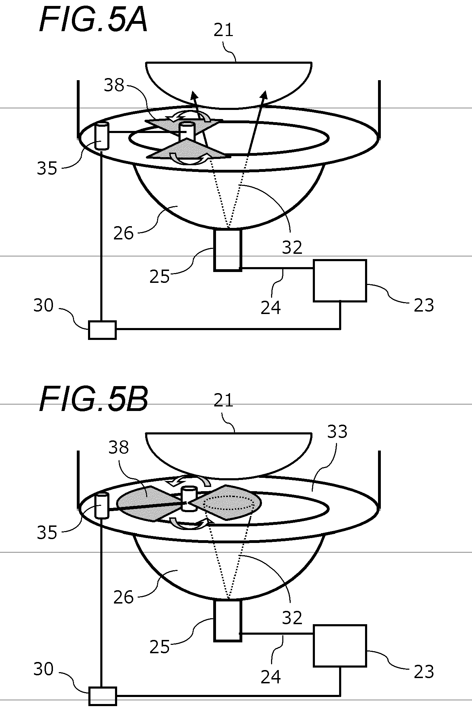

In addition, a configuration shown in FIGS. 5A and 5B uses an impeller blade-type acoustic wave generating member 38 made of a material (such as a black polyethylene sheet) which absorbs light. The acoustic wave generating member 38 is rotatably provided and is movable between a position (FIG. 5A) which is retreated from the optical path between the irradiating optical system 25 and the object 21 and a position (FIG. 5B) which blocks the optical path. By controlling the motor 35 to rotate the acoustic wave generating member 38, the switching unit 30 can switch the optical path between an open state and a blocked state. Alternatively, the switching unit 30 may continuously switch between the first mode and the second mode at high speed by controlling the pulse irradiation timing of the Ti:Sa laser light source 23 and the rotation timing of the acoustic wave generating member 38.

<Second practical example> (not claimed)

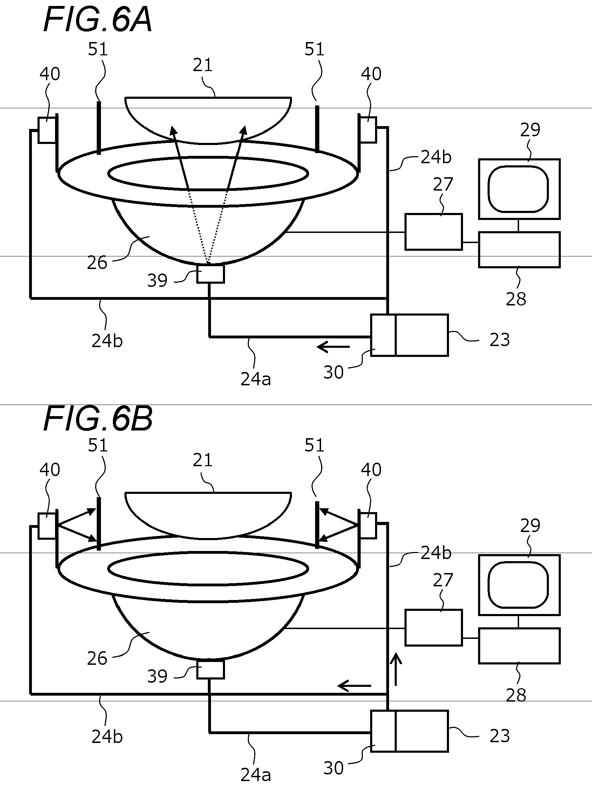

FIGS. 6A and 6B are system configuration diagrams of a photoacoustic measuring apparatus according to a second practical example. FIG. 6A shows a state of the first mode (PAT measurement) and FIG. 6B shows a state of the second mode (PAUS measurement). Moreover, the same components as those in the first practical example will be denoted by the same reference numerals and a description thereof will be omitted.

The photoacoustic measuring apparatus according to the second practical example includes an object irradiating optical system 39 (a first irradiating unit) which irradiates the object 21 with light and an acoustic wave generating member irradiating optical system 40 (a second irradiating unit) which irradiates an acoustic wave generating member 51 with light. The switching unit 30 switches between guiding light emitted from the Ti:Sa laser light source 23 to the object irradiating optical system 39 and guiding the light to the acoustic wave generating member irradiating optical system 40.

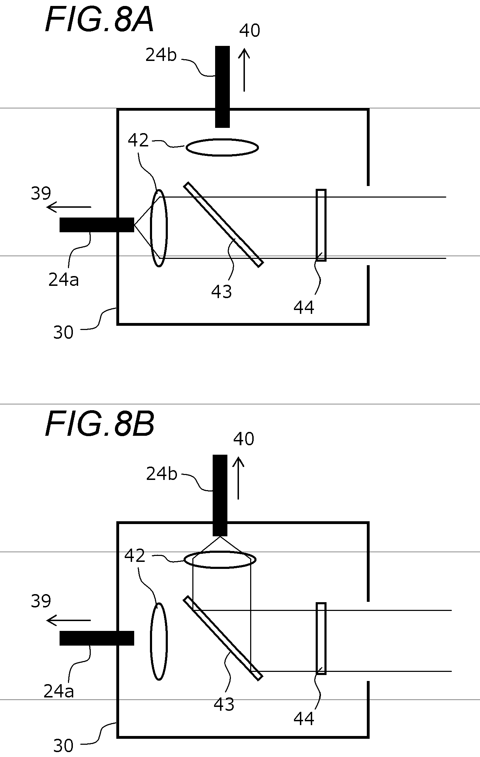

FIGS. 8A and 8B show an example of an internal configuration of the switching unit 30. The switching unit 30 includes a convex lens 42, a reflection type polarizing plate 43, and a Pockels cell 44. The Pockels cell 44 is an element which controls a polarization direction of light. A liquid crystal may be used in place of the Pockels cell. The reflection type polarizing plate 43 is an element which transmits light in a first polarization direction and which reflects light in a second polarization direction that is perpendicular to the first polarization direction.

Light in the first polarization direction is emitted from a Ti:Sa laser light source. In the first mode, light from the laser light source is transmitted through the Pockels cell 44 and, as shown in FIG. 8A, transmitted through the reflection type polarizing plate 43, condensed by the convex lens 42, and subsequently enters an optical fiber 24a connected to the object irradiating optical system 39. On the other hand, in the second mode, voltage is applied to the Pockels cell 44 to rotate a polarization direction of light by 90 degrees. As a result, as shown in FIG. 8B, the light is reflected by the reflection type polarizing plate 43, condensed by the convex lens 42, and subsequently enters an optical fiber 24b connected to the acoustic wave generating member irradiating optical system 40.

(Modifications) (not claimed)

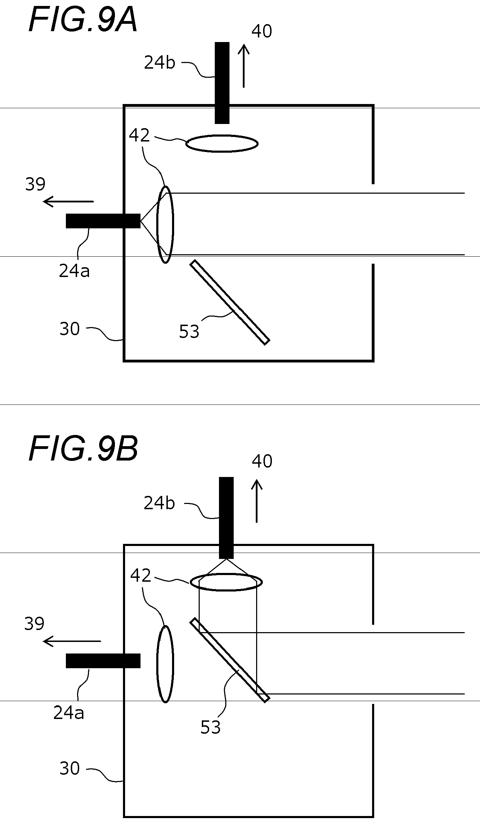

FIGS. 9A and 9B show another configuration example of the switching unit 30. While the switching unit 30 switches the polarization direction in FIGS. 8A and 8B, in FIGS. 9A and 9B, the switching unit 30 switches the optical path with a mirror 53. Specifically, in the first mode, as shown in FIG. 9A, the switching unit 30 guides light from a laser light source to the optical fiber 24a connected to the object irradiating optical system 39 by causing the mirror 53 to retreat from the optical path. In the second mode, as shown in FIG. 9B, the switching unit 30 guides light from the laser light source to the optical fiber 24b connected to the acoustic wave generating member irradiating optical system 40 by inserting the mirror 53 to the optical path. Moreover, configurations of the switching unit 30 are not limited thereto and the switching unit 30 need only switch between guiding light to the object irradiating optical system 39 and guiding light to the acoustic wave generating member irradiating optical system 40.

In addition, while the present practical example is provided with two irradiating units including the first irradiating unit and the second irradiating unit, since irradiation need only be performed at a position where an object is irradiated and at a position where an acoustic wave generating member is irradiated, one irradiating unit may be provided with a moving mechanism and may be movable to a position where an object is irradiated and to a position where an acoustic wave generating member is irradiated. Any mechanism may be used as long as irradiation of the object and irradiation of the acoustic wave generating member can be respectively performed in an isolated manner.

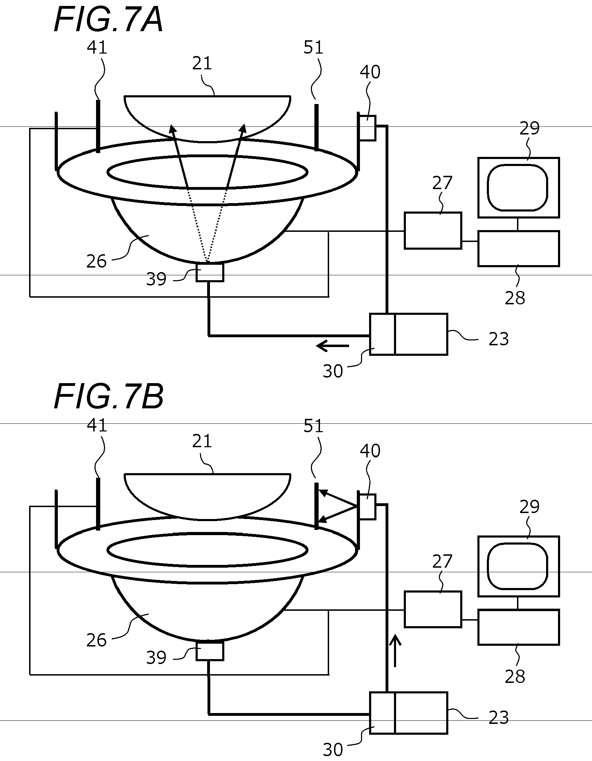

Furthermore, as shown in FIGS. 7A and 7B, a photoacoustic measuring apparatus may be provided with a transmitted acoustic wave receiving probe 41 which receives an acoustic wave generated by the acoustic wave generating member 51 and transmitted inside the object 21. The acoustic wave generating member 51 and the transmitted acoustic wave receiving probe 41 may be arranged opposing each other across the object 21. In addition, the work station PC 28 may calculate a sound velocity distribution inside the object 21 using a signal received by the transmitted acoustic wave receiving probe 41. Furthermore, the acoustic wave generating member 51 and the transmitted acoustic wave receiving probe 41 may have a mechanism that enables rotation around the object 21 and may measure transmitted acoustic waves at a plurality of locations while rotating.

<Third practical example> (not claimed)

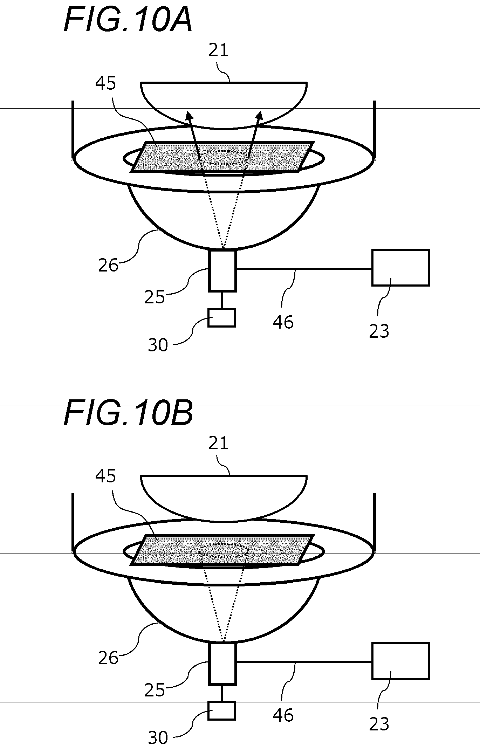

FIGS. 10A and 10B are system configuration diagrams of a photoacoustic measuring apparatus according to a third practical example. FIG. 10A shows a state of the first mode (PAT measurement) and FIG. 10B shows a state of the second mode (PAUS measurement). Moreover, the same components as those in the first practical example will be denoted by the same reference numerals and a description thereof will be omitted.

The third practical example uses an acoustic wave generating member 45 made of an absorbing-type polarizing plate which transmits light in a first polarization direction and absorbs light in a second polarization direction that is perpendicular to the first polarization direction. The switching unit 30 is an apparatus which switches the polarization direction of light from the Ti:Sa laser light source 23. Specifically, by controlling application of voltage to a Pockels cell or a liquid crystal provided in the irradiating optical system 25, the switching unit 30 switches between outputting light in the first polarization direction from the laser light source 23 without modification and outputting light in the second polarization direction by rotating the polarization direction by 90 degrees. Furthermore, a waveguide 46 between the laser light source 23 and the irradiating optical system 25 is a waveguide for spatial propagation in order to hold the state of polarized light transmitted from the laser light source 23.

In the first mode, light in the first polarization direction is irradiated from the irradiating optical system 25. As shown in FIG. 10A, the light in the first polarization direction is transmitted through the acoustic wave generating member 45 and irradiates the object 21. On the other hand, in the second mode, the switching unit 30 applies voltage to the Pockels cell in the irradiating optical system 25 to rotate the polarization direction of light by 90 degrees. As a result, as shown in FIG. 10B, light in the second polarization direction is completely absorbed by the acoustic wave generating member 45. Even with such a configuration, switching between the first mode and the second mode can be realized.

<Fourth practical example> (not claimed)

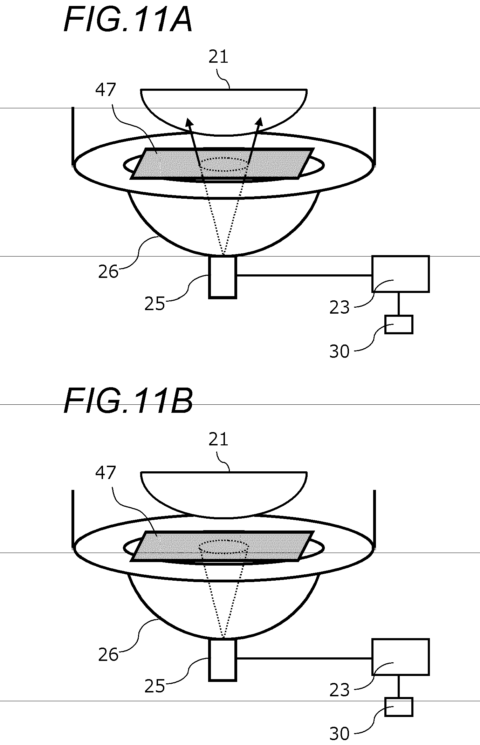

FIGS. 11A and 11B are system configuration diagrams of a photoacoustic measuring apparatus according to a fourth practical example. FIG. 11A shows a state of the first mode (PAT measurement) and FIG. 11B shows a state of the second mode (PAUS measurement). Moreover, the same components as those in the first practical example will be denoted by the same reference numerals and a description thereof will be omitted.

The fourth practical example uses an acoustic wave generating member 47 made of a wavelength-selective absorbing film which transmits light with a first wavelength and absorbs light with a second wavelength which differs from the first wavelength. In the present practical example, the used acoustic wave generating member 47 characteristically transmits light with a wavelength of 797 nm and absorbs light with a wavelength of 532 nm. The switching unit 30 is an apparatus which switches the wavelength of light emitted from the light source 23.

In the first mode, the switching unit 30 switches the wavelength of light emitted from the light source 23 to 797 nm. As shown in FIG. 11A, light with a wavelength of 797 nm is transmitted through the acoustic wave generating member 47 and irradiates the object 21. On the other hand, in the second mode, the switching unit 30 switches the wavelength of light emitted from the light source 23 to 532 nm. As a result, as shown in FIG. 11B, light with a wavelength of 532 nm is completely absorbed by the acoustic wave generating member 47. Even with such a configuration, switching between the first mode and the second mode can be realized.

Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment (s) . The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD) ™), a flash memory device, a memory card, and the like.

While the present invention has been described with reference to exemplary embodiments, it is to be understood that the invention is not limited to the disclosed exemplary embodiments, but by the scope of the following claims.

An object information acquiring apparatus includes: an acoustic wave generating member which absorbs light and generates an acoustic wave; an irradiating unit which irradiates an object or the acoustic wave generating member with light; a detector which detects an acoustic wave propagating from the object; a signal processing unit which generates object information that is information of the inside of the object, based on a signal output from the detector; and a switching unit which performs switching between a first mode in which a first acoustic wave generated inside the object due to irradiation of the light is detected by the detector and a second mode in which a second acoustic wave generated by the acoustic wave generating member due to irradiation of the light and having propagated inside the object is detected by the detector.

## Claims

1. An object information acquiring apparatus, comprising:

    an acoustic wave generating member (4) configured to absorb light (3) and to generate an acoustic wave (10);
    an irradiating unit (2) configured to irradiate an object (5) or the acoustic wave generating member (4) with light (3);
    a detector (6) configured to detect an acoustic wave propagating from the object;
    a signal processing unit (7) configured to generate object information that is information of the inside of the object, based on a signal output from the detector; and
    a switching unit (8) configured to perform switching between a first mode in which a first acoustic wave generated inside the object (5) due to irradiation of the light (3) is detected by the detector (6) and a second mode in which a second acoustic wave generated by the acoustic wave generating member (4) due to irradiation of the light (3) and having propagated inside the object (5) is detected by the detector (6),
    **characterized in that**
    the acoustic wave generating member (4) is movable to a first position retreated from an optical path between the irradiating unit (2) and the object (5) and to a second position that blocks the optical path, and
    the switching unit (8) is configured to switch a position of the acoustic wave generating member (4) so that the acoustic wave generating member (4) moves to the first position in the first mode and the acoustic wave generating member (4) moves to the second position in the second mode.

2. An object information acquiring apparatus, comprising:

    an acoustic wave generating member (4) configured to absorb light (3) and to generate an acoustic wave (10);
    an irradiating unit (2) configured to irradiate an object (5) or the acoustic wave generating member (4) with light (3);
    a detector (6) configured to detect an acoustic wave propagating from the object;
    a signal processing unit (7) configured to generate object information that is information of the inside of the object, based on a signal output from the detector; and
    a switching unit (8) configured to perform switching between a first mode in which a first acoustic wave generated inside the object (5) due to irradiation of the light (3) is detected by the detector (6) and a second mode in which a second acoustic wave generated by the acoustic wave generating member (4) due to irradiation of the light (3) and having propagated inside the object (5) is detected by the detector (6),
    **characterized in that**
    the irradiating unit (2) is movable to a first position where the object (5) is irradiated with the light (3) and to a second position where the acoustic wave generating member (4) is irradiated with the light (3), and
    the switching unit is configured to switch a position of the irradiating unit (2) so that the irradiating unit (2) moves to the first position in the first mode and the irradiating unit (2) moves to the second position in the second mode.

3. The object information acquiring apparatus according to any one of claims 1 to 2, wherein the acoustic wave generating member is a member with a sheet shape or a flat plate shape.

4. A control method for an object information acquiring apparatus including an acoustic wave generating member (4) which absorbs light (3) and generates an acoustic wave (10), an irradiating unit (2) which irradiates an object (5) or the acoustic wave generating member (4) with light (3), a detector (6) which detects an acoustic wave propagating from the object (5), and a signal processing unit (7) which generates object information that is information of the inside of the object (5), based on a signal output from the detector (6),

the control method comprising:

a first measurement step (S2) of detecting a first acoustic wave generated inside the object (5) due to irradiation of the light (3) with the detector (6) and acquiring a first detected signal; a second measurement step (S5) of detecting a second acoustic wave generated by the acoustic wave generating member (4) due to irradiation of the light and having propagated inside the object (5) with the detector (6) and acquiring a second detected signal;

**characterized by**

a switching step (S4) of moving the acoustic wave generating member (4) in the first measurement step (S2) to a first position retreated from an optical path between the irradiating unit (2) and the object (5) so that only the object (5) is irradiated with the light (3) and of moving the acoustic wave generating member (4) in the second measurement step (S5) to a second position that blocks the optical path so that only the acoustic wave generating member (4) is irradiated with the light (3).

5. A control method for an object information acquiring apparatus including an acoustic wave generating member (4) which absorbs light (3) and generates an acoustic wave (10), an irradiating unit (2) which irradiates an object (5) or the acoustic wave generating member (4) with light (3), a detector (6) which detects an acoustic wave propagating from the object, and a signal processing unit (7) which generates object information that is information of the inside of the object (5), based on a signal output from the detector (6),

the control method comprising:

a first measurement step (S2) of detecting a first acoustic wave generated inside the object (5) due to irradiation of the light (3) with the detector (6) and acquiring a first detected signal; a second measurement step (S5) of detecting a second acoustic wave generated by the acoustic wave generating member (4) due to irradiation of the light (3) and having propagated inside the object (5) with the detector (6) and acquiring a second detected signal;

**characterized by**

a switching step (S4) of moving the irradiating unit (2) to a first position so that only the object (5) is irradiated with the light (3) when performing the first measurement step (S2), and to a second position so that only the acoustic wave generating member (4) is irradiated with the light (3) when performing the second measurement step (S5).

**Patentansprüche**

1. Objektinformationserhaltevorrichtung mit:

einem Akustische-Welleerzeugungsbauteil (4), das konfiguriert ist, Licht (3) zu absorbieren und eine akustische Welle (10) zu erzeugen; einer Bestrahlungseinheit (2), die konfiguriert ist, ein Objekt (5) oder das Akustische-Welleerzeugungsbauteil (4) mit Licht (3) zu bestrahlen; einem Detektor (6), der konfiguriert ist, eine akustische Welle, die von dem Objekt propagiert, zu erfassen; einer Signalverarbeitungseinheit (7), die konfiguriert ist, eine Objektinformation, die eine Information über das Innere des Objekts ist, basierend auf einem Signal, das von dem Detektor ausgegeben wird, zu erzeugen; und einer Schalteinheit (8), die konfiguriert ist, ein Schalten zwischen einem ersten Modus, in dem eine erste akustische Welle innerhalb des Objekts (5) aufgrund einer Bestrahlung mit dem Licht (3) durch den Detektor (6) erfasst wird, und einem zweiten Modus, in dem eine zweite akustische Welle, die durch das Akustische-Welleerzeugungsbauteil (4) aufgrund einer Bestrahlung mit dem Licht (4) erzeugt wird und die innerhalb des Objekts (5) propagiert ist, durch den Detektor (6) erfasst wird, durchzuführen, **dadurch gekennzeichnet, dass**

das Akustische-Welleerzeugungsbauteil (4) zu einer ersten Position, die aus einem optischen Pfad zwischen der Bestrahlungseinheit (2) und dem Objekt (5) zurückgezogen ist, und einer zweiten Position, die den optischen Pfad blockiert, bewegbar ist, und die Schalteinheit (8) konfiguriert ist, eine Position des Akustische-Welleerzeugungsbauteil (4) so zu schalten, dass sich das Akustische-Welleerzeugungsbauteil (4) zu der ersten Position in dem ersten Modus bewegt, und sich das Akustische-Welleerzeugungsbauteil (4) zu der zweiten Position in dem zweiten Modus bewegt.

2. Objektinformationserhaltevorrichtung mit:

einem Akustische-Welleerzeugungsbauteil (4), das konfiguriert ist, Licht (3) zu absorbieren und eine akustische Welle (10) zu erzeugen;

einer Bestrahlungseinheit (2), die konfiguriert ist, ein Objekt (5) oder das Akustische-Welleerzeugungsbauteil (4) mit Licht (3) zu bestrahlen;

einem Detektor (6), der konfiguriert ist, eine akustische Welle, die von dem Objekt propagiert, zu erfassen;

einer Signalverarbeitungseinheit (7), die konfiguriert ist, eine Objektinformation, die eine Information über das Innere des Objekts ist, basierend auf einem Signal, das von dem mit Detektor ausgegeben wird, zu erzeugen; und

einer Schalteinheit (8), die konfiguriert ist, ein Schalten zwischen einem ersten Modus, in dem meine erste akustische Welle, die innerhalb des Objekts (5) aufgrund einer Bestrahlung mit dem Licht (3) durch den Detektor (6) erfasst wird, und einem zweiten Modus, in dem eine zweite akustische Welle, die durch das Akustische-Welleerzeugungsbauteil (4) aufgrund einer Bestrahlung mit dem Licht (3) erzeugt wird und durch das Innere des Objekts (5) propagiert ist, durch den Detektor (6) erfasst wird, durchzuführen,

**dadurch gekennzeichnet, dass**

die Bestrahlungseinheit (2) zu einer ersten Position, an der das Objekt (5) mit dem Licht (3) bestrahlt wird, und zu einer zweiten Position, bei der das Akustische-Welleerzeugungsbauteil (4) mit dem Licht (3) bestrahlt wird, bewegbar ist, und

die Schalteinheit konfiguriert ist, eine Position der Bestrahlungseinheit (2) so umzuschalten, dass sich die Bestrahlungseinheit (2) zu der ersten Position in dem ersten Modus bewegt und sich die Bestrahlungseinheit (2) zu der zweiten Position in dem zweiten Modus bewegt.

3. Objektinformationserhaltevorrichtung nach einem der Ansprüche 1 bis 2, wobei

das Akustische-Welleerzeugungsbauteil ein Bauteil mit einer Plattenform oder einer Form einer flachen Platte ist.

4. Steuerungsverfahren für eine Objektinformationerhaltevorrichtung mit einem Akustische-Welleerzeugungsbauteil (4), das Licht (3) absorbiert und eine akustische Welle (10) erzeugt, einer Bestrahlungseinheit (2), die ein Objekt (5) oder das Akustische-Welleerzeugungsbauteil (4) mit Licht (3) bestrahlt, einem Detektor (6), der eine akustische Welle, die von dem Objekt (5) propagiert, erfasst, und einer Signalverarbeitungseinheit (7), die eine Objektinformation, die eine Information über das Innere des Objekts (5) ist, basierend auf einem Signal, das von den Detektor (6) ausgegeben wird, erzeugt, wobei das Steuerungsverfahren aufweist:

einen ersten Messschritt (S2) des Erfassens einer ersten akustischen Welle, die innerhalb des Objekts (5) aufgrund einer Bestrahlung mit dem Licht (3) erzeugt wird, mit dem Detektor (6) und des Erhaltens eines ersten erfassten Signals;

einen zweiten Messschritt (S5) des Erfassens einer zweiten akustischen Welle, die durch das Akustische-Welleerzeugungsbauteil (4) aufgrund einer Bestrahlung mit dem Licht erzeugt wird und die durch das Innere des Objekts (5) propagiert ist, mit dem Detektor (6) und des Erhaltens eines zweiten erfassten Signals;

**gekennzeichnet durch**

einen Schaltschritt (S4) des Bewegens des Akustische-Welleerzeugungsbauteils (4) in dem ersten Messschritt (S2) zu einer ersten Position, die von einem optischen Pfad zwischen der Bestrahlungseinheit (2) und dem Objekt (5) zurückgezogen ist, sodass nur das Objekt (5) mit dem Licht (3) bestrahlt wird, und des Bewegens des Akustische-Welleerzeugungsbauteils (4) in dem zweiten Messschritt (S5) zu einer zweiten Position, die den optischen Pfad blockiert, sodass nur das Akustische-Welleerzeugungsbauteil (4) mit dem Licht bestrahlt wird.

5. Steuerungsverfahren für eine Objektinformationserhaltevorrichtung mit einem Akustische-Welleerzeugungsbauteil (4), das Licht (3) absorbiert und eine akustische Welle (10) erzeugt, einer Bestrahlungseinheit (2), die ein Objekt (5) oder das Akustische-Welleerzeugungsbauteil (4) mit Licht (3) bestrahlt, einem Detektor (6), der eine akustische Welle, die von dem Objekt (5) propagiert, erfasst, und einer Signalverarbeitungseinheit (7), die eine Objektinformation, die eine Information über das Innere des Objekts (5) ist, basierend auf einem Signal, das von den Detektor (6) ausgegeben wird, erzeugt, wobei das Steuerungsverfahren aufweist:

einen ersten Messschritt (S2) des Erfassens einer ersten akustischen Welle, die innerhalb des Objekts (5) aufgrund einer Bestrahlung mit dem Licht (3) erzeugt wird, mit dem Detektor (6) und des Erhaltens eines ersten erfassten Signals;

einen zweiten Messschritt (S5) des Erfassens einer zweiten akustischen Welle, die durch das Akustische-Welleerzeugungsbauteil (4) aufgrund einer Bestrahlung mit dem Licht erzeugt wird und die durch das Innere des Objekts (5) propagiert ist, mit dem Detektor (6) und des Erhaltens eines zweiten erfassten Signals;

**gekennzeichnet durch**

einen Schaltschritt (S4) des Bewegens der Bestrahlungseinheit (2) zu einer ersten Position, sodass nur das Objekt (5) mit dem Licht (3) bestrahlt wird, wenn der erste Messschritt (S2) durchgeführt wird, und zu einer zweiten Position, sodass nur das Akustische-Welleerzeugungsbauteil (4) mit dem Licht (3) bestrahlt wird, wenn der zweite Messschritt (S5) durchgeführt wird.

**Revendications**

1. Appareil d'acquisition d'informations d'objet, comprenant :

   un élément de génération d'onde acoustique (4) configuré pour absorber de la lumière (3) et pour générer une onde acoustique (10) ;
   une unité d'exposition à un rayonnement (2) configurée pour exposer un objet (5) ou l'élément de génération d'onde acoustique (4) à un rayonnement de la lumière (3) ;
   un détecteur (6) configuré pour détecter une onde acoustique se propageant à partir de l'objet ;
   une unité de traitement de signal (7) configurée pour générer des informations d'objet qui sont des informations de l'intérieur de l'objet, sur la base d'un signal délivré par le détecteur ; et
   une unité de commutation (8) configurée pour effectuer une commutation entre un premier mode dans lequel une première onde acoustique générée à l'intérieur de l'objet (5) du fait de l'exposition au rayonnement de la lumière (3) est détectée par le détecteur (6) et un second mode dans lequel une seconde onde acoustique générée par l'élément de génération d'onde acoustique (4) du fait de l'exposition à un rayonnement de la lumière (3) et s'étant propagée à l'intérieur de l'objet (5) est détectée par le détecteur (6),
   **caractérisé en ce que**
   l'élément de génération d'onde acoustique (4) peut être amené à une première position en retrait d'un trajet optique entre l'unité d'exposition à un rayonnement (2) et l'objet (5) et à une seconde position qui bloque le trajet optique, et l'unité de commutation (8) est configurée pour commuter une position de l'élément de génération d'onde acoustique (4) de sorte que l'élément de génération d'onde acoustique (4) soit amené à la première position dans le premier mode et que l'élément de génération d'onde acoustique (4) soit amené à la seconde position dans le second mode.

2. Appareil d'acquisition d'informations d'objet, comprenant :

   un élément de génération d'onde acoustique (4) configuré pour absorber de la lumière (3) et pour générer une onde acoustique (10) ;
   une unité d'exposition à un rayonnement (2) configurée pour exposer un objet (5) ou l'élément de génération d'onde acoustique (4) à un rayonnement de lumière (3) ;
   un détecteur (6) configuré pour détecter une onde acoustique se propageant à partir de l'objet ;
   une unité de traitement de signal (7) configurée pour générer des informations d'objet qui sont des informations de l'intérieur de l'objet, sur la base d'un signal délivré par le détecteur ; et
   une unité de commutation (8) configurée pour effectuer une commutation entre un premier mode dans lequel une première onde acoustique générée à l'intérieur de l'objet (5) du fait de l'exposition à un rayonnement de la lumière (3) est détectée par le détecteur (6) et un second mode dans lequel une seconde onde acoustique générée par l'élément de génération d'onde acoustique (4) du fait de l'exposition à un rayonnement de la lumière (3) et se propageant à l'intérieur de l'objet (5) est détectée par le détecteur (6),
   **caractérisé en ce que**
   l'unité d'exposition à un rayonnement (2) peut être amenée à une première position à laquelle l'objet (5) est exposé à un rayonnement de la lumière (3) et à une seconde position à laquelle l'élément de génération d'onde acoustique (4) est exposé à un rayonnement de la lumière (3), et
   l'unité de commutation est configurée pour commuter une position de l'unité d'exposition à un rayonnement (2) de sorte que l'unité d'exposition à un rayonnement (2) soit amenée à la première position dans le premier mode et que l'unité d'exposition à un rayonnement (2) soit amenée à la seconde position dans le second mode.

3. Appareil d'acquisition d'informations d'objet selon l'une quelconque des revendications 1 et 2, dans lequel l'élément de génération d'onde acoustique est un élément en forme de feuille ou en forme de plaque plate.

4. Procédé de commande d'un appareil d'acquisition d'informations d'objet comprenant un élément de génération d'onde acoustique (4) qui absorbe de la lumière (3) et qui génère une onde acoustique (10), une unité d'exposition à un rayonnement (2) qui expose un objet (5) ou l'élément de génération d'onde acoustique (4) à un rayonnement de la lumière (3), un détecteur (6) qui détecte une onde acoustique se propageant à partir de l'objet (5), et une unité de traitement de signal (7) qui génère des informations d'objet qui sont des informations de l'intérieur de l'objet (5), sur la base d'un signal délivré par le détecteur (6), le procédé de commande comprenant :

   une première étape de mesure (S2) consistant à détecter une première onde acoustique générée à l'intérieur de l'objet (5) du fait de l'exposition à un rayonnement de la lumière (3) au moyen du détecteur (6) et à acquérir un premier signal détecté ;

une seconde étape de mesure (S5) consistant à détecter une seconde onde acoustique générée par l'élément de génération d'onde acoustique (4) du fait de l'exposition à un rayonnement de la lumière et s'étant propagée à l'intérieur de l'objet (5) au moyen du détecteur (6) et à acquérir un second signal détecté ;

**caractérisée par**

une étape de commutation (S4) consistant à amener l'élément de génération d'onde acoustique (4), à la première étape de mesure (S2), à une première position en retrait d'un trajet optique entre l'unité d'exposition à un rayonnement (2) et l'objet (5) de sorte que seul l'objet (5) soit exposé à un rayonnement de la lumière (3) et à amener l'élément de génération d'onde acoustique (4), à la seconde étape de mesure (S5), à une seconde position qui bloque le trajet optique de sorte que seul l'élément de génération d'onde acoustique (4) soit exposé à un rayonnement de la lumière (3).

5. Procédé de commande d'un appareil d'acquisition d'informations d'objet comprenant un élément de génération d'onde acoustique (4) qui absorbe de la lumière (3) et qui génère une onde acoustique (10), une unité d'exposition à un rayonnement (2) qui expose un objet (5) ou l'élément de génération d'onde acoustique (4) à un rayonnement de lumière (3), un détecteur (6) qui détecte une onde acoustique se propageant à partir de l'objet, et une unité de traitement de signal (7) qui génère des informations d'objet qui sont des informations de l'intérieur de l'objet (5), sur la base d'un signal délivré par le détecteur (6), le procédé comprenant :

une première étape de mesure (S2) consistant à détecter une première onde acoustique générée à l'intérieur de l'objet (5) du fait de l'exposition à un rayonnement de la lumière (3) au moyen du détecteur (6) et à acquérir un premier signal détecté ;

une seconde étape de mesure (S5) consistant à détecter une seconde onde acoustique générée par l'élément de génération d'onde acoustique (4) du fait de l'exposition à un rayonnement de la lumière (3) et s'étant propagée à l'intérieur de l'objet (5) au moyen du détecteur (6) et à acquérir un second signal détecté ;

**caractérisé par**

une étape de commutation (S4) consistant à amener l'unité d'exposition à un rayonnement (2) à une première position de sorte que seul l'objet (5) soit exposé à un rayonnement de la lumière (3) lors de l'exécution de la première étape de mesure (S2), et à une seconde position de sorte que seul l'élément de génération d'onde acoustique (4) soit exposé à un rayonnement de la lumière (3) lors de l'exécution de la seconde étape de mesure (S5).

# FIG.1A

# FIG.1B

# FIG.2

START

S 1 — IRRADIATE OBJECT WITH PULSED LIGHT

S 2 — RECEIVE ACOUSTIC WAVE AND CONVERT INTO FIRST DETECTED SIGNAL

S 3 — CALCULATE FIRST OBJECT INFORMATION USING FIRST DETECTED SIGNAL

S 4 — PERFORM SWITCHING SO THAT PULSED LIGHT IS ABSORBED BY ACOUSTIC WAVE GENERATING MEMBER

S 5 — IRRADIATE ACOUSTIC WAVE GENERATING MEMBER WITH PULSED LIGHT

S 6 — RECEIVE ACOUSTIC WAVE AND CONVERT INTO SECOND DETECTED SIGNAL

S 7 — CALCULATE SECOND OBJECT INFORMATION USING SECOND DETECTED SIGNAL

END

## FIG.3A

## FIG.3B

## FIG.4A

## FIG.4B

*FIG.5A*

*FIG.5B*

## *FIG.6A*

## *FIG.6B*

FIG.7A

FIG.7B

## FIG.8A

## FIG.8B

## FIG.9A

## FIG.9B

## FIG.10A

## FIG.10B

## FIG.11A

## FIG.11B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100041987 A
- US 20130190595 A1
- US 20130190594 A1
- US 20050131289 A1
- US 20130031982 A1
- US 20130245420 A1

**Non-patent literature cited in the description**

- **THOMAS FELIX FEHM ; XOSE LUIS DEAN-BEN ; DANIEL RAZANSKY.** Hybrid optoacoustic and ultrasound imaging in three dimensions and real time by optical excitation of a passive element. *Proc. of SPIE,* vol. 9323